# EUROPÄISCHE PATENTSCHRIFT

(11) **EP 2 173 405 B1**
(45) Veröffentlichungstag und Bekanntmachung des Hinweises auf die Patenterteilung: **16.09.2015**
(21) Anmeldenummer: 08784992.3
(22) Anmeldetag: 23.07.2008
(51) Int. Cl.: A61M 1/16, A61M 1/36

(54) **HERZ-LUNGEN-MASCHINE**
HEART-LUNG MACHINE
MACHINE COEUR-POUMON

(30) Priorität: 09.08.2007 DE 102007037755
(43) Veröffentlichungstag der Anmeldung: 14.04.2010
(73) Patentinhaber: Rheinisch-Westfälisch-Technische Hochschule Aachen, 52062 Aachen (DE)
(72) Erfinder: VÁZQUEZ-JIMÉNEZ, Jaime, 52072 Aachen (DE); SCHNÖRING, Heike, 4728 Hergenrath (BE); SCHMITZ-RODE, Thomas, 52070 Aachen (DE); STEINSEIFER, Ulrich, 4730 Hauset (BE); ARENS, Jutta, 52072 Aachen (DE)
(74) Vertreter: Cohausz Hannig Borkowski Wißgott
(86) Internationale Anmeldenummer: PCT/EP2008/006037
(87) Internationale Veröffentlichungsnummer: WO 2009/018920

(56) Entgegenhaltungen:
- EP-A- 0 167 162
- EP-A- 0 766 974
- WO-A-97/16213
- DE-A1-102005 029 682
- US-A- 5 167 921
- US-A- 5 270 005

## Beschreibung

Die Erfindung betrifft eine Herz-Lungen-Maschine umfassend ein Kardiotomiereservoir und einen Oxygenator, in welchem eine Blutpumpe angeordnet ist, wobei das Kardiotomiereservoir direkt am Oxygenator angeordnet ist und der Blutausgang des Kardiotomiereservoirs in den Bluteingang der Blutpumpe mündet und das Kardiotomiereservoir unterteilt ist in ein oberes Notfallvolumen und ein unteres Arbeitsvolumen, wobei das Arbeitsvolumen ausgebildet ist durch einen Kanal, dessen oberes Ende in das Notfallvolumen und dessen unteres Ende in den Bluteingang der Blutpumpe mündet und der in einem transparenten unteren Abschnitt des Kardiotomiereservoirs liegt, welcher oberhalb und ausserhalb des Oxygenators angeordnet ist, so dass der Füllstand des Blutes in diesem Kanal visuell erfassbar ist.

Herz-Lungen-Maschinen sind im Stand der Technik bekannt, z.B. aus der US 5,270,005 A und der US 5,167,921 A und werden üblicherweise bei Operationen am offenen, nicht schlagenden Herzen eingesetzt, um den Gasaustausch der Lunge und die Pumpleistung des Herzens zu übernehmen. Hierfür wird über eine entsprechende Kanülierung das venöse Blut eines Patienten über einen Schlauch in das Kardiotomiereservoir geleitet, insbesondere hierbei gefiltert und entschäumt und aus dem Kardiotomiereservoir einem Oxygenator zugeführt.

Dabei ist es im Stand der Technik bekannt, einerseits Kardiotomiereservoir und Oxygenator voneinander zu trennen und durch Schlauchverbindungen den Übergang des Blutes zwischen diesen Bauteilen erfolgen zu lassen oder aber andererseits auch das Kardiotomiereservoir direkt am Oxygenator anzuordnen, so dass der Blutausgang des Kardiotomiereservoirs direkt in den Bluteingang der Blutpumpe mündet, die innerhalb des Oxygenators angeordnet sein kann.

Ein solcher Oxygenator umfasst dabei Faserbündel aus vielen mikroporösen, semipermeablen Einzelfasern, die vom Blut umströmt werden, während sie gleichzeitig von einem geeigneten Gasgemisch durchströmt werden. Hierdurch findet eine Anreicherung des Blutes mit Sauerstoff und eine Elimination des Kohlendioxids im Blut statt. Das so arterialisierte Blut wird vom Oxygenator sodann üblicherweise über einen Schlauch zurück in den Patienten geleitet.

Ein während einer Operation oder auch nach einer Operation erzeugter extrakorporaler Blutkreislauf, der durch eine solche Herz-Lungen-Maschine führt, kann häufig zu akuten, systemischen entzündlichen Reaktionen sowie auch zur Schädigung der Gerinnungssysteme führen. Patienten können dabei postoperativ an Fieber und Fehlfunktionen verschiedener Organsysteme in unterschiedlichem Ausmaß leiden, wobei insbesondere Neugeborene in besonderem Maße gefährdet sind.

Ein besonderes Problem von Herz-Lungen-Maschinen ist es dabei, dass diese ein gewisses Arbeitsvolumen an Blut benötigen, welches üblicherweise größer ist als das gesamte Blutvolumen von Neugeborenen, so dass hier regelmäßig Fremdblut zum Einsatz kommen muss.

Zusätzlich entwickeln besonders neugeborene Kinder häufig entzündliche Reaktionen, die durch den Ausschluß der Lungenperfusion, Myokardischämie und Reperfusion, den Kontakt zwischen Blut und Fremdkörperoberflächen und die mechanische Verletzung der Blutbestandteile zustande kommen.

Um Risiken gerade bei Neugeborenen, jedoch auch bei erwachsenen Patienten zu minimieren, besteht daher eine allgemeine Tendenz, die Volumina von Herz-Lungen-Maschinen zu verringern. Dabei waren die bisherigen Ansätze im Wesentlichen derart, dass die überwiegend von dem Einsatz bei Erwachsenen bekannten Herz-Lungen-Maschine hinsichtlich ihrer Einzelkomponenten lediglich verkleinert wurden, was jedoch problematisch ist, da einer solchen Verkleinerung Grenzen gesetzt sind, z. B. durch die benötigte Gasaustauschfläche, die benötigten Schlauchverbindungen der Komponenten untereinander etc.

Aufgabe der Erfindung ist es, eine Herz-Lungen-Maschine der eingangsgenannten Art bereitzustellen, welche ein verbessertes Verhältnis von Gasaustauschfläche zu dem Arbeitsvolumen aufweist, um derartige Maschinen mit einer besseren Effizienz und geringeren Risiken bei Erwachsenen und insbesondere auch bei Neugeborenen zum Einsatz bringen zu können. Insbesondere ist es dabei die Aufgabe, das Arbeitsvolumen einer solchen Herz-Lungen-Maschine möglichst weit zu verringern.

Erfindungsgemäß wird diese Aufgabe dadurch gelöst, dass bei einer Herz-Lungen-Maschine der eingangs genannten Art innerhalb des Kardiotomiereservoirs ein Wärmetauscherelement angeordnet ist, welches sich zumindest teilweise bis in den Kanal erstreckt.

Bei der Erfindung ist das Kardiotomiereservoir unterteilt in ein oberes Notfallvolumen und ein unteres Arbeitsvolumen, wobei das Arbeitsvolumen ausgebildet ist durch einen Kanal, dessen oberes Ende in das (größere) Notfallvolumen und dessen unteres Ende in den Bluteingang der Blutpumpe mündet und der in einem transparenten unteren Abschnitt des Kardiotomiereservoirs liegt, welcher oberhalb und außerhalb des Oxygenators angeordnet ist, so dass der Füllstand des Blutes in diesem Kanal visuell erfassbar ist.

Diesen Merkmalen liegt der Kerngedanke zugrunde, dass oberhalb des Bluteingangs der Blutpumpe eine gewisse Mindesthöhe an Blutsäule anstehen muss, um zu verhindern, dass Umgebungsluft in die Blutpumpe eingezogen wird. Aus diesem Grund muss das Kardiotomiereservoir für den sicheren Betrieb einer Herz-Lungen-Maschine bis zu einer gewissen Mindestfüllhöhe aufgefüllt sein mit Blut, um einen solchen Betrieb zu gewährleisten.

Dabei ist es der Gedanke, die Füllhöhe mit Blut in dem Kardiotomiereservoir in einem gegenüber dem Notfallvolumen querschnittsreduzierten Bereich zu realisieren, so dass es vorgesehen ist, das Kardiotomiereservoir erfindungsgemäß zu unterteilen in das besagte obere Notfallvolumen und untere Arbeitsvolumen, wobei das untere Arbeitsvolumen vorgesehen ist, um diesen Mindestfüllstand an Blutsäule zu erzielen.

Dabei wird das Arbeitsvolumen durch einen Kanal der eingangs genannten Art realisiert, dessen Querschnitt geringer ist als der Querschnitt des Notfallvolumens, so dass die benötigte Füllhöhe mit einem geringen Blutvolumen erreicht werden kann und wodurch somit das benötigte Arbeitsvolumen einer erfindungsgemäßen Herz-Lungen-Maschine gegenüber üblichen Herz-Lungen-Maschinen deutlich reduziert werden kann, bei denen das Kardiotomiereservoir üblicherweise einen festen Querschnitt aufweist und am unteren Ende von diesem Querschnitt unmittelbar in den Bluteingang der Blutpumpe übergeht.

Dabei kann es beispielsweise vorgesehen sein, dass der Kanal, der zur Ausbildung des Arbeitsvolumens vorgesehen ist, einen Querschnitt aufweist, der zumindest in seinem unteren dem Bluteingang der Pumpe zuweisenden Bereich - um den Faktor 5 bis 20, bevorzugt 14 bis 17 mal geringer ist als der Querschnitt des darüber liegenden Notfallvolumens. So kann bei einer derartigen Anordnung dafür Sorge getragen werden, dass die benötigte Blutflüssigkeitssäule oberhalb des Bluteingangs ansteht, ohne dass hierfür ein großes Volumen benötigt wird. Weiterhin stellt das oberhalb des Kanals vorgesehene Notfallvolumen dennoch ein derart großes Gesamtvolumen des Kardiotomiereservoirs dar, dass die Möglichkeit besteht, große Blutvolumina aus einem Patienten aufzunehmen, wenn dies im Rahmen einer Operation notwendig werden sollte.

Dabei ist es gemäß der weiterhin vorgesehenen Ausgestaltung der Herz-Lungen-Maschine vorgesehen, dass der eingangs genannte Kanal in einem transparenten unteren Abschnitt des Kardiotomiereservoirs angeordnet ist, welcher oberhalb und außerhalb des Oxygenators angeordnet ist. Dies bietet den besonderen Vorteil, dass der transparente Kanal und die darin stehende Blutflüssigkeitssäule durch die die Herz-Lungen-Maschine bedienende Kardiotechniker jederzeit visuell erfassbar ist und so der Füllstand des Blutes in diesem Kanal jederzeit überprüfbar und gegebenenfalls korrigierbar ist.

Für die Ausbildung eines Kanals als Arbeitsvolumen unterhalb des Notfallvolumens eines Kardiotomiereservoirs kann dabei grundsätzlich jegliche Formgestaltung vorgesehen sein, die geeignet ist, eine Volumenreduzierung gegenüber dem Notfallvolumen zu erzielen. So besteht beispielsweise die Möglichkeit, dass der Kanal sich vom oberen Ende, wo er in das Notfallvolumen mündet, bis zum unteren Ende, wo er in den Bluteingang mündet, nach unten hin verjüngt, z. B. vom Querschnitt des Notfallvolumens bis zum Querschnitt des Bluteingangs der Blutpumpe.

In einer anderen Ausgestaltung kann es auch vorgesehen sein, dass der Querschnitt des Kanals über die gesamte Länge des Kanals gleich bleibt. Die Querschnittsform ist hierbei grundsätzlich irrelevant, jedoch kann die runde Querschnittsform technisch bevorzugt sein.

Um eine ausreichende Füllstandshöhe des Blutes innerhalb des Kanals zu erzielen, kann es bei diesen Ausführungen bevorzugt vorgesehen sein, dass die Höhe/Länge dieses Kanals im Vergleich zur Höhe des Notfallvolumens bevorzugt im Wesentlichen gleich ist oder dass die Höhe des Kanals zumindest dem 0,5 bis 1-fachen der Höhe des Notfallvolumens entspricht.

Hierdurch wird insgesamt erreicht, dass die Höhe des Kanals ausreichend groß ist, um die Veränderung des Füllstandes der Blutflüssigkeitssäule innerhalb dieses Kanals bequem mit dem Auge visuell erfassen zu können.

In einer bevorzugten Ausführung einer erfindungsgemäßen Herz-Lungen-Maschine kann es dabei vorgesehen sein, dass das Kardiotomiereservoir und der Oxygenator voneinander lösbare Baueinheiten bilden. So kann hierdurch beispielsweise gewährleistet sein, dass z.B. der Blutpumpenantrieb wieder verwendet werden kann, wohingegen blutführende Teile nach einer Benutzung entsorgt werden.

Durch die Ausbildung als voneinander lösbare Baueinheiten können diese voneinander getrennt und somit zum Teil entsorgt werden. Die Verbindung der beiden Einheiten untereinander kann dabei bevorzugt durch einen Schiebemechanismus erzielt werden, bei dem es vorgesehen sein kann, dass das untere Ende des Kardiotomiereservoirs auf das obere Ende des Oxygenators aufgesetzt wird und durch Bewegung eines translatierbaren Schiebers, der an einem der beiden Bauteile vorgesehen ist, die Verbindung untereinander zu erzielen. Dabei kann im Übergangsbereich zwischen diesen beiden Bauteilen z. B. um die Öffnung des Bluteingangs der Blutpumpe ein Dichtring angeordnet sein.

Bei einer Herz-Lungen-Maschine der erfindungsgemäßen Art kann es ebenso vorgesehen sein wie bei den Herz-Lungen-Maschinen, wie sie im Stand der Technik bekannt sind, einen Wärmetauscher einzusetzen, um das Blut zu konditionieren, d. h. je nach Anwendungsfall aufzuwärmen oder auch gezielt abzukühlen, je nach Erfordernis bei einer Operation.

Hierbei ist es in den im Stand der Technik bekannten Herz-Lungen-Maschinen vorgesehen, einen Wärmetauscher innerhalb eines Oxygenators oder um einen Oxygenator herum anzuordnen, um eine solche Konditionierung vorzunehmen. Dies führt jedoch immer zwingend dazu, dass weitere Volumenführende Bauteile geschaffen werden müssen, um einen entsprechenden Blutkontakt zu realisieren und somit einen Wärmeübertritt zwischen Blut und Wärmetauscher zu ermöglichen. Die im Stand der Technik bekannten Maßnahmen zur Realisierung eines Wärmetauschers innerhalb einer Herz-Lungen-Maschine führen somit immer zu einer Volumenvergrößerung und damit zu einer Vergrößerung der damit einhergehenden Risiken für den Patienten aufgrund der erheblich vergrößerten Blutverdünnung bzw. des erhöhten Fremdblutbedarfes.

Gemäß der Erfindung ist es vorgesehen, dass ein Wärmetauscherelement innerhalb des Kardiotomiereservoirs angeordnet wird, welches sich zumindest teilweise bis in den Kanal hinein erstreckt. Hierdurch wird erfindungsgemäß erzielt, dass aus dem Kanal, der das Arbeitsvolumen bildet bzw. im Wesentlichen den Mindestfüllstand an Blut definiert, Blut durch das Wärmetauscherelement verdrängt wird, so dass sich hierdurch eine weitere Reduktion des Blutvolumens innerhalb dieses Arbeitsvolumens ergibt, ohne dass hierdurch die Füllstandshöhe beeinträchtigt wird. So bleibt der gesamte mögliche Füllstand an Blut innerhalb des Kanals weiterhin durch die gesamte Länge des Kanals bestimmt, wobei sich jedoch das Arbeitsvolumen des Kanals durch das zumindest teilweise bis in den Kanal sich hineinerstreckende Wärmetauscherelement reduziert.

So kann es in einer besonders bevorzugten Ausführung vorgesehen sein, dass sich das Wärmetauscherelement über die gesamte Länge des Kanals erstreckt, um so eine maximale Blutverdrängung aus dem Arbeitsvolumen zu erzielen. Hierbei kann es bevorzugt vorgesehen sein, dass zumindest der Bereich des Wärmetauscherelementes, der sich in den Kanal erstreckt, bevorzugt das Wärmetauscherelement über seine gesamte Länge, zumindest im Wesentlichen in seinem äußeren Querschnitt an den inneren Querschnitt des Kanals angepasst ist.

Auch durch diese Querschnittsanpassung kann dabei der Effekt der Blutverdrängung aus dem Arbeitsvolumen optimiert werden. Beispielsweise kann es dabei vorgesehen sein, dass der innere Querschnitt des Kanals rund ist, ebenso wie der äußere Querschnitt des Wärmetauscherelementes.

Hierbei kann es vorgesehen sein, dass gerade dann, wenn sich das Wärmetauscherelement über die gesamte Länge des Kanals erstreckt, das untere Ende des Wärmetauscherelementes unmittelbar vor oder in oder auch hinter der Mündungsöffnung des Bluteinlasses der Blutpumpe bzw. des Oxygenators liegt. Hierbei kann das untere Ende des Wärmetauscherelementes leicht verjüngend ausgebildet sein und gegebenenfalls durch die Mündungsöffnung des Bluteinlasses der Blutpumpe hindurchragen. Hierdurch wird im Wesentlichen bei einem ansonsten kreisförmigen Querschnitt des Bluteinlasses ein ringförmiger Querschnitt des Bluteinlasses erzielt und damit selbst noch im Bereich der Eingangsmündung im Bluteingang der Blutpumpe eine Volumenreduktion hervorgerufen.

Ein Wärmetauscherelement, welches bei der erfindungsgemäßen Herz-Lungen-Maschine zum Einsatz kommen kann, kann unterschiedliche Formen aufweisen. Gemäß einer bevorzugten erfindungsgemäßen Ausgestaltung kann es beispielsweise vorgesehen sein, dass das Wärmetauscherelement als doppelwandiges Rohr ausgebildet ist mit einer koaxialen Hin- und Rückführung der Wärmetauscherflüssigkeit. Hierbei kann bevorzugt das Wärmetauscherelement ein zentrales Zuflussrohr aufweisen, um welches herum zur Ausbildung der doppelwandigen Rohrkonstruktion ein im Durchmesser größeres Abflussrohr angeordnet ist. Im unteren Bereich des Wärmetauscherelementes kann sich der Übergangsbereich zwischen diesen beiden Rohren befinden, so dass die Zu- und Abführung der Wärmetauscherflüssigkeit oberhalb und außerhalb des Kardiotomiereservoirs erfolgen kann.

In einer anderen Ausführung kann es auch vorgesehen sein, dass das Wärmetauscherelement ein zentrales Zuflussrohr aufweist und ein sich schraubenförmig um das Zuflussrohr herumwindendes Abflussrohr. Bei beiden Ausführungen kann der äußere Querschnitt des Wärmetauscherelementes im Wesentlichen kreisförmig ausgebildet sein.

Weiterhin kann die Führung der Wärmetauscherflüssigkeit bevorzugt derart gewählt sein, dass die Wärmetauscherflüssigkeit im äußeren Bereich des Wärmetauscherelementes dem Blut im Kanal entgegenströmt Hierdurch kann ein Wärmetausch im Gegenstromprinzip realisiert werden.

In einer weiterhin bevorzugten Weiterbildung kann es dabei auch vorgesehen sein, dass das Wärmetauscherelement auf seiner Außenseite ergänzend Lamellen aufweist, die sich in Längsrichtung des Wärmetauscherelementes erstrecken, insbesondere bei der Bauform, bei der das Wärmetauscherelement als doppelwandiges Rohr ausgebildet ist. Dabei wird die Querschnittsgestaltung des Wärmetauscherelementes bevorzugt derart gewählt, dass der Querschnitt, wie er im Wesentlichen durch die radial äußeren Kanten der Lamellen definiert ist, im Wesentlichen an den Querschnitt des Kanals angepasst ist, beispielsweise also im Wesentlichen rund ist.

In einer weiterhin bevorzugten Ausführung kann es vorgesehen sein, dass im Kardiotomiereservoir oberhalb des Kanals im Notfallvolumen ein Blutfilter angeordnet ist, durch den das Blut in das Kardiotomiereservoir einströmt. Ein solcher Blutfilter kann zum Filtern und Entschäumen des einströmenden Blutes verwendet werden und in einer bevorzugten Ausführung kann es dabei vorgesehen sein, dass Blutfilter und Wärmetauscherelement nebeneinander angeordnet sind, insbesondere in einem Kardiotomiereservoir, dessen Notfallvolumen einen bevorzugt von der Kreisform abweichenden Querschnitt hat.

Beispielsweise kann der Querschnitt des Notfallvolumens des erfindungsgemäßen Kardiotomiereservoirs im Wesentlichen herzförmig ausgestaltet sein, wobei die Mittenachsen von Wärmetauscherelement und Blutfilter bevorzugt in der Symmetrieebene des herzförmig ausgebildeten Reservoirs liegen.

In einer weiterhin besonders bevorzugten Ausgestaltung kann es auch vorgesehen sein, dass der Bluteingang der Blutpumpe umschaltbar entweder vom Blutausgang des Kardiotomiereservoirs oder unter Umgehung des Kardiotomiereservoirs direkt aus einem zweiten alternativen Blutausgang mit Blut beaufschlagbar ist. So kann eine derart ausgebildete erfindungsgemäße Herz-Lungen-Maschine auch für eine sogenannte ECMO-Anwendung (Extra-CorporaleMembran-Oxygenierung) eingesetzt werden.

Bei einer solchen Anwendung kann der Einsatz eines Kardiotomiereservoirs an einer Herz-Lungen-Maschine entbehrlich sein, so dass es demnach vorgesehen sein kann, bei einer erfindungsgemäßen Herz-Lungen-Maschine das

Kardiotomiereservoir zumindest fluidtechnisch abzukoppeln, bevorzugt bei einer solchen ECMO-Anwendung sogar als Bauteil abzunehmen.

In einer Weiterbildung kann es dabei vorgesehen sein, dass zwischen dem Kardiotomiereservoir und dem Oxygenator ein Zwischenelement einsetzbar ist, in welchem eine Umschaltung zwischen den Blutausgängen bewirkbar ist. Allgemein kann es bei allen Ausführungen der Umschaltung bevorzugt vorgesehen sein, dass eine automatische Umschaltung erfolgt, sobald das Kardiotomiereservoir abgekoppelt wird vom Oxygenator.

So kann eine ECMO-Anwendung in unmittelbarem Anschluss an die Anwendung mit der gesamten Herz-Lungen-Maschine erfolgen, ohne Wechsel des Oxygenators und ohne dass ein erneutes Primen von anderweitigen Komponenten erforderlich ist.

Eine Herz-Lungen-Maschine der eingangs genannten erfindungsgemäßen Art hat gegenüber den im Stand der Technik bekannten Herz-Lungen-Maschinen den besonderen Vorteil, dass hier nicht nur die an sich bekannten Bauteile oder Baugruppen in ihren Ausmaßen zu kleineren Baugrößen skaliert werden, sondern insbesondere, dass das Verhältnis von Membranoberfläche im Oxygenator zu dem mindestens benötigten Arbeitsvolumen der Herz-Lungen-Maschine in Richtung eines größeren Verhältnisses optimiert werden kann. Hierdurch erschließen sich gerade mit der erfindungsgemäßen Herz-Lungen-Maschine Anwendungsgebiete bei Neugeborenen, da durch die erfindungsgemäße Ausführung des kanalartig ausgebildeten Arbeitsvolumens und bevorzugt eines darin angeordneten Wärmetauscherelementes das benötigte Füll- bzw. Arbeitsvolumen kleiner als 125 Milliliter sein kann, bevorzugt kann sogar ein Füllvolumen kleiner gleich 75 Milliliter realisiert sein, jeweils bereits unter Berücksichtigung der weiteren Blutvolumina in den Blutzufuhr- und Abfuhrschläuchen sowie des Arbeitsvolumens des Kardiotomiereservoirs. Derart kleine Volumina können dabei insbesondere bei Membranoberflächen des Oxygenators von 0,21 bzw. 0,36 Quadratmeter realisiert werden.
Ein Ausführungsbeispiel der Erfindung ist in den nachfolgenden Figuren dargestellt. Es zeigen:
- Figur 1: ein Kardiotomiereservoir gemäß der Erfindung
- Figur 2: eine gesamte Herz-Lungen-Maschine mit einem Kardiotomiereservoir gemäß Figur 1

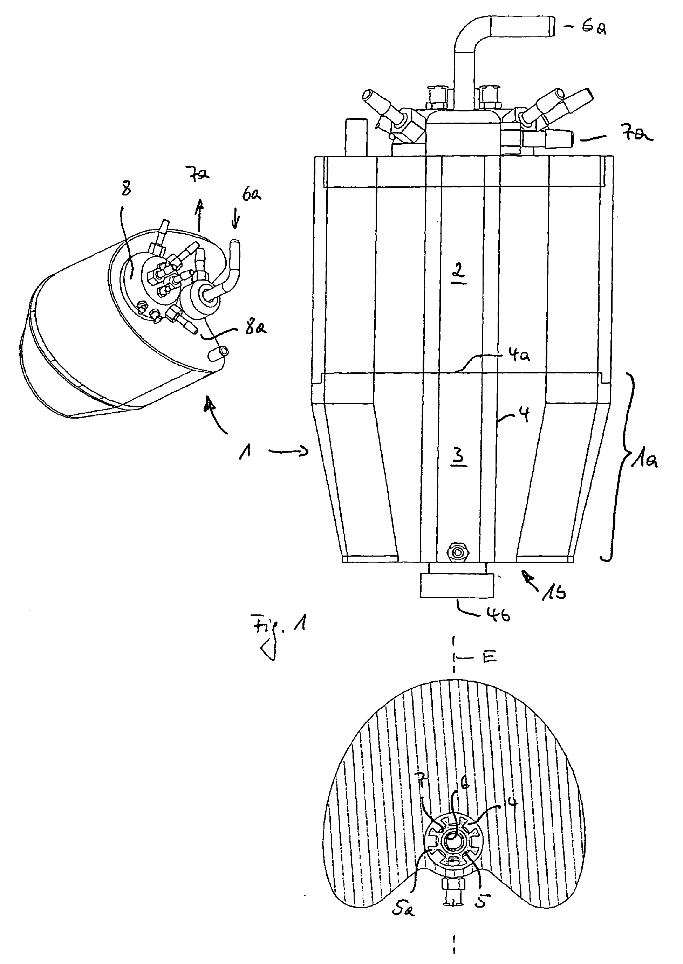

Die Figur 1 zeigt in zwei Schnitt- und einer perspektivischen Darstellung ein Kardiotomiereservoir, wie es an einer erfindungsgemäßen Herz-Lungen-Maschine zum Einsatz kommen kann.

Das Kardiotomiereservoir 1 weist hierbei ein oberes Notfallvolumen 2 und ein unteres Arbeitsvolumen 3 auf. Das untere Arbeitsvolumen 3 ist dabei im Wesentlichen definiert durch einen Kanal 4, der mit seinem oberen Ende 4a in das Notfallvolumen mündet und mit seinem unteren Ende 4b in den Bluteingang einer Blutpumpe, die z. B. hier zentrisch in einem Oxygenator angeordnet sein kann.

Die untere Schnittdarstellung zeigt dabei, dass der Kanal 4 ausgebildet ist mit einem kreisförmigen, gleich bleibenden Querschnitt über die gesamte Länge des Kanals 4 zwischen den beiden Enden 4a und 4b, wobei hier dargestellt ist, dass in diesem Kanal 4 ein Wärmetauscherelement 5 angeordnet ist, welches auf seiner Außenseite radial vorspringende Lamellen 5a trägt und wobei weiterhin dieses Wärmetauscherelement doppelwandig ausgebildet ist mit einem inneren Rohr 6, welches zur Zuführung von Wärmetauscherflüssigkeit vorgesehen ist über den oberhalb und außerhalb des Kardiotomiereservoirs liegenden Zufluss 6a.

Das äußere Rohr 7 trägt dabei unmittelbar die Lamellen 5a des Wärmetauscherelementes, wobei in dem Zwischenbereich zwischen dem inneren Rohr 6 und dem äußeren Rohr 7 die Wärmetauscherflüssigkeit wieder nach oben strömt und aus dem Ausgang 7a oberhalb des Kardiotomiereservoirs austritt.

Zumindest der untere Bereich 1a des Kardiotomiereservoirs 1, bevorzugt der gesamte Reservoirbereich ist aus einem transparenten Kunststoff ausgebildet, um die Höhe des Blutpegels innerhalb des Kanals 3/4 visuell beobachten zu können.

Ersichtlich wird hier gerade bei Betrachtung der unteren Schnittdarstellung, dass durch die Einführung des Wärmetauscherelementes 5 in den Kanal4 neben dem ohnehin reduzierten Volumen des Kanals weiterhin das Blutvolumen vermindert wird, da das Wärmetauscherelement 5 signifikant Blut aus dem Kana14 verdrängt.

Neben dem Wärmetauscherelement kann es hier auch vorgesehen sein, einen Filter 8 im Kardiotomiereservoir vorzusehen, wobei Wärmetauscherelement und Filter nebeneinander angeordnet sind, insbesondere dabei in einer Symmetrieebene E der im Wesentlichen herzförmig gestalteten Querschnittsform des Kardiotomiereservoirs 1 liegen. Der Filter 8 weist dabei beispielsweise einen Blutzufluss 8a sowie auch andere Zuführungen für z. B. Medikamentengaben oder ähnliches auf.

Das Kardiotomiereservoir 1 der hier dargestellten Art weist an seinem unteren Ende 1 b die Möglichkeit auf, an ein oberes Ende eines Oxygenators 9 angekoppelt zu werden.

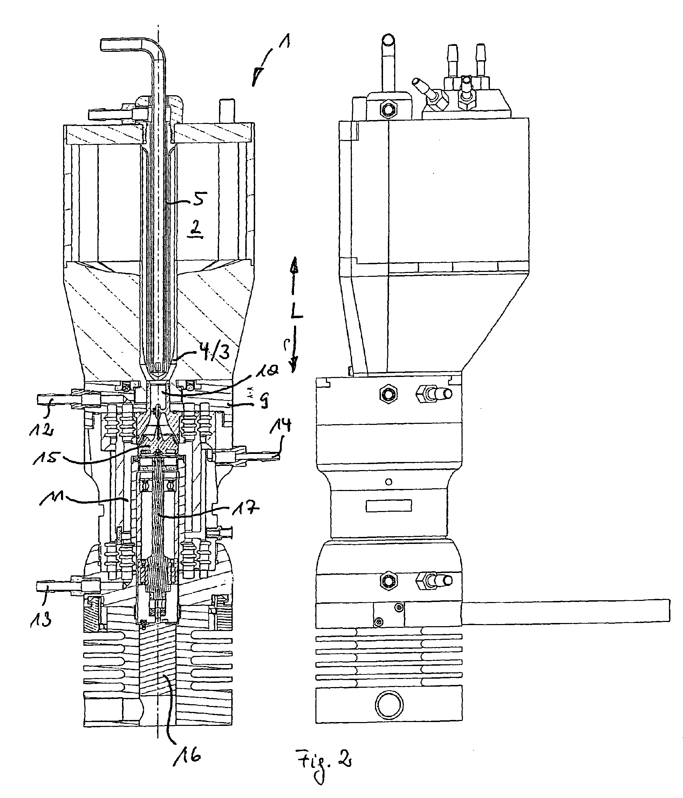

Die Figur 2 zeigt eine durch die Ankopplung realisierte Herz-Lungen-Maschine der erfindungsgemäßen Art, bei der das Kardiotomiereservoir 1, wie es in der Figur 1 beschrieben ist, an das obere Ende eines Oxygenators 9 angekoppelt ist. Der Oxygenator 9 weist dabei einen Bluteingang 10 auf, der im Wesentlichen durch die Mündungsöffnung einer zentrisch im Oxygenator 9 angeordneten Blutpumpe gegeben ist, die hier als Rotorpumpe ausgebildet ist.

Durch die zentrische Mündungsöffnung des Rotors wird das Blut aus der Bluteingangsöffnung 10 aus dem Arbeitsvolumen 3 bzw. dem Kanal 4 des Kardiotomiereservoirs 1 angesaugt und an den semipermeablen Membranen 11, die koaxial um die Pumpenmechanik angeordnet sind, vorbeigefördert. Dabei sind die semipermeablen Membranen 11 durch Gaseinlass 12 und Gasauslass 13 mit einem geeigneten Gas zur Oxygenierung des Blutes durchströmt. Das oxygenierte Blut kann sodann aus dem Blutauslass 14 aus der erfindungsgemäßen Herz-Lungen-Maschine austreten und z. B. durch einen Schlauch dem Patienten zugeführt werden.

Der Antrieb der Blutpumpe, die im Wesentlichen hier durch den drehbar gelagerten Rotor 15 gegeben ist, kann hierdurch eine magnetische Wirkverbindung bzw. magnetische Kupplung zum Rotor 15 erfolgen. Hierzu kann ein Oxygenator der erfindungsgemäßen Art eine zentrische Bohrung aufweisen, in die ein Antrieb 16 mit einer angetriebenen eingekapselten Welle 17 eingesetzt werden kann, die an ihrem oberen Ende eine entsprechende Magnetkupplung zur Mitbewegung des Rotors durch magnetische Ankopplung aufweist. So kann zumindest ein Antrieb 16 jederzeit wieder verwendet werden.

Nicht dargestellt ist in dieser Zeichnung die Möglichkeit, zwischen dem oberen Ende des Oxygenators 9 und dem unteren Ende des Kardiotomiereservoirs 1 ein Zwischenelement einzufügen, mit dem eine Umschaltung bewirkt werden kann, nämlich um zum einen das Blut aus dem Blutauslass des Kanals 4 zu beziehen oder nach Umschaltung alternativ aus einem anderen Blutauslass, der z. B. direkt durch einen Schlauch mit einem Patienten verbunden sein kann.

Mit Bezug auf die Figur 2 ist hier erkennbar, dass über die Länge L, die im Wesentlichen der Länge des Kanals 4 bzw. der Höhe des Arbeitsvolumens entspricht, der Blutfüllstand im Kardiotomiereservoir bzw. dessen unteren Arbeitsvolumens 3 visuell beobachtet werden kann, da sich dieser Bereich des Kardiotomiereservoirs bzw. der Kanal oberhalb und außerhalb des Oxygenators 9 angeordnet ist und dabei weiterhin von transparentem Material umgeben ist, so dass die Blutsäule unmittelbar von einem behandelnden Arzt beobachtet werden kann. Der Füllstand des Blutes innerhalb des Kanals 4 kann somit jederzeit korrigiert werden.

Bezüglich sämtlicher Ausführungen ist festzustellen, dass die in Verbindung mit einer Ausführung genannten technischen Merkmale nicht nur bei der spezifischen Ausführung eingesetzt werden können, sondern auch bei den jeweils anderen Ausführungen. Sämtliche offenbarten technischen Merkmale dieser Efindungsbeschreibung sind als erfindungswesentlich einzustufen und beliebig miteinander kombinierbar oder in Alleinstellung einsetzbar.

## Patentansprüche

1. Herz-Lungen-Maschine umfassend ein Kardiotomiereservoir und einen Oxygenator, in welchem eine Blutpumpe angeordnet ist, wobei das Kardiotomiereservoir direkt am Oxygenator angeordnet ist und der Blutausgang des Kardiotomiereservoirs in den Bluteingang der Blutpumpe mündet und das Kardiotomiereservoir (1) unterteilt ist in ein oberes Notfallvolumen (2) und ein unteres Arbeitsvolumen (3), wobei das Arbeitsvolumen (3) ausgebildet ist durch einen Kanal (4), dessen oberes Ende (4a) in das Notfallvolumen (2) und dessen unteres Ende (4b) in den Bluteingang (10) der Blutpumpe mündet und der in einem transparenten unteren Abschnitt (1a) des Kardiotomiereservoirs (1) liegt, welcher oberhalb und ausserhalb des Oxygenators (9) angeordnet ist, so dass der Füllstand des Blutes in diesem Kanal (4) visuell erfassbar ist, **dadurch gekennzeichnet, dass** innerhalb des Kardiotomiereservoirs (1) ein Wärmetauscherelement (5) angeordnet ist, welches sich zumindest teilweise bis in den Kanal (4) erstreckt.

2. Maschine nach Anspruch 1, **dadurch gekennzeichnet, dass** das Kardiotomiereservoir (1) und der Oxygenator (9) voneinander lösbare Baueinheiten bilden, insbesondere die durch einen Schiebemechanismus miteinander verbindbar sind.

3. Maschine nach einem der vorherigen Ansprüche, **dadurch gekennzeichnet, dass** zumindest der Bereich des Wärmetauscherelements (5), der sich in den Kanal (4) erstreckt, bevorzugt das Wärmetauscherelement (5) über die gesamte Länge zumindest im Wesentlichen in seinem äußeren Querschnitt an den inneren Querschnitt des Kanals (4) angepasst ist.

4. Maschine nach einem der vorherigen Ansprüche, **dadurch gekennzeichnet, dass** das untere Ende des Wärmetauscherelements (5) unmittelbar vor oder in der Mündungsöffnung des Bluteinlasses (1 0) der Blutpumpe / des Oxygenators (9) liegt.

5. Maschine nach einem der vorherigen Ansprüche, **dadurch gekennzeichnet, dass** das Wärmetauscherelement (5) als doppelwandiges Rohr ausgebildet ist mit einer koaxialen Hin- (6) und Rückführung (7) der Wärmetauscherflüssigkeit.

6. Maschine nach einem der vorherigen Ansprüche, **dadurch gekennzeichnet, dass** das Wärmetauscherelement (5) ein zentrales Zuflussrohr und ein sich schraubenförmig um das Zuflussrohr herumwindendes Abflussrohr aufweist.

7. Maschine nach Anspruch 5 oder 6, **dadurch gekennzeichnet, dass** die Führung der Wärmetauscherflüssigkeit derart ist, dass die Wärmetauscherflüssigkeit im äußeren Bereich des Wärmetauscherelements dem Blut im Kanal (4) entgegenströmt.

8. Maschine nach einem der vorherigen Ansprüche, **dadurch gekennzeichnet, dass** das Wärmetauscherelement (5) auf seiner Außenseite Lamellen (5a) aufweist, die sich in Längsrichtung des Wärmtauscherelementes (5) erstrecken.

9. Maschine nach einem der vorherigen Ansprüche, **dadurch gekennzeichnet, dass** im Kardiotomiereservoir (1) oberhalb des Kanals (4) im Notfallvolumen (2) ein Blutfilter (8) angeordnet ist, durch den das Blut in das Kardiotomiereservoir (1) einströmt.

10. Maschine nach Anspruch 9, **dadurch gekennzeichnet, dass** Blutfilter (8) und Wärmetauscherelement (5) nebeneinander angeordnet sind.

11. Maschine nach einem der vorherigen Ansprüche, **dadurch gekennzeichnet, dass** der Bluteingang (1 0) der Blutpumpe umschaltbar entweder vom Blutausgang des Kardiotomiereservoirs (1) oder unter Umgehung des Kardiotomiereservoirs (1) direkt aus einem zweiten Blutausgang mit Blut beaufschlagbar ist.

12. Maschine nach Anspruch 11, **dadurch gekennzeichnet, dass** zwischen dem Kardiotomiereservoir (1) und dem Oxygenator (9) ein Zwischenelement setzbar ist, in welchem eine Umschaltung zwischen den Blutausgängen bewirkbar ist.

13. Maschine nach einem der vorherigen Ansprüche 11 oder 12, **dadurch gekennzeichnet, dass** eine automatische Umschaltung erfolgt, bei Abkopplung des Kardiotomiereservoirs (1).

14. Maschine nach einem der vorherigen Ansprüche, **dadurch gekennzeichnet, dass** sie bei einer Membranoberfläche des Qxygenators (9) von 0,21 bis 0,36 qm ein Füllvolumen kleiner als 125 ml bevorzugt kleiner als 75 ml aufweist unter Einberechnung der Blutzufuhr und -abfuhrschläuche sowie des Arbeitsvolumens des Kardiotomiereservoirs (1).

## Claims

1. Heart-lung machine comprising a cardiotomy reservoir and an oxygenator in which a blood pump is arranged, wherein the cardiotomy reservoir is arranged directly on the oxygenator and the blood outlet of the cardiotomy reservoir opens into the blood inlet of the blood pump, and the cardiotomy reservoir (1) is divided into an upper emergency volume (2) and a lower working volume (3), wherein the working volume (3) is formed by a channel (4) whose upper end (4a) opens into the emergency volume (2) and whose lower end (4b) opens into the blood inlet (10) of the blood pump and which lies in a transparent lower portion (1a) of the cardiotomy reservoir (1) arranged above and outside the oxygenator (9), such that the filling level of the blood in this channel (4) can be ascertained visually, **characterized in that** a heat exchanger element (5) is arranged inside the cardiotomy reservoir (1) and extends at least partially into the channel (4).

2. Machine according to Claim 1, **characterized in that** the cardiotomy reservoir (1) and the oxygenator (9) form structural units which are detachable from each other, in particular which are connectable to each other by a slide mechanism.

3. Machine according to one of the preceding claims, **characterized in that** at least the area of the heat exchanger element (5) extending into the channel (4), preferably the entire length of the heat exchanger element (5), is adapted at least substantially in its outer cross section to the inner cross section of the channel (4).

4. Machine according to one of the preceding claims, **characterized in that** the lower end of the heat exchanger element (5) lies directly in front of or within the mouth of the blood inlet (10) of the blood pump/of the oxygenator (9).

5. Machine according to one of the preceding claims, **characterized in that** the heat exchanger element (5) is designed as a double-walled pipe with a coaxial supply (6) and return (7) of the heat exchanger liquid.

6. Machine according to one of the preceding claims, **characterized in that** the heat exchanger element (5) has a central inflow pipe, and an outflow pipe winding helically around the inflow pipe.

7. Machine according to Claim 5 or 6, **characterized in that** the guiding of the heat exchanger liquid is such that the heat exchanger liquid in the outer area of the heat exchanger element flows counter to the blood in the channel (4).

8. Machine according to one of the preceding claims, **characterized in that** the heat exchanger element (5) has, on its outside, slats (5a) extending in the longitudinal direction of the heat exchanger element (5).

9. Machine according to one of the preceding claims, **characterized in that** a blood filter (8) is arranged in the cardiotomy reservoir (1) above the channel (4) in the emergency volume (2), through which blood filter (8) the blood flows into the cardiotomy reservoir (1).

10. Machine according to Claim 9, **characterized in that** blood filter (8) and heat exchanger element (5) are arranged next to each other.

11. Machine according to one of the preceding claims, **characterized in that** the blood inlet (10) of the blood pump can be supplied with blood either from the blood outlet of the cardiotomy reservoir (1) or, bypassing the cardiotomy reservoir (1), directly from a second blood outlet.

12. Machine according to Claim 11, **characterized in that** an intermediate element, in which a switch can be effected between the blood outlets, can be placed between the cardiotomy reservoir (1) and the oxygenator (9).

13. Machine according to either of Claims 11 and 12 **characterized in that** an automatic switch takes place upon uncoupling of the cardiotomy reservoir (1).

14. Machine according to one of the preceding claims, **characterized in that**, with the oxygenator (9) having a membrane surface area of 0.21 to 0.36 m², the machine has a filling volume of less than 125 ml, preferably less than 75 ml, taking into account the blood inflow and outflow hoses and the working volume of the cardiotomy reservoir (1).

## Revendications

1. Machine cardiopulmonaire comprenant un réservoir de cardiotomie et un oxygénateur dans lequel une pompe à sang est disposée, le réservoir de cardiotomie étant disposé directement au niveau de l'oxygénateur et la sortie de sang du réservoir de cardiotomie débouchant dans l'entrée de sang de la pompe à sang et le réservoir de cardiotomie (1) étant divisé en un volume d'urgence supérieur (2) et un volume opératoire inférieur (3), le volume opératoire (3) étant constitué par un canal (4) dont l'extrémité supérieure (4a) débouche dans le volume d'urgence (2) et dont l'extrémité inférieure (4b) débouche dans l'entrée de sang (10) de la pompe à sang et qui se situe dans une section inférieure transparente (1a) du réservoir de cardiotomie (1) qui est disposé au-dessus et à l'extérieur de l'oxygénateur (9), de sorte que le niveau de remplissage sanguin de ce canal (4) est détectable visuellement, **caractérisée en ce qu'**est disposé dans le réservoir de cardiotomie (1) un élément échangeur de chaleur (5) qui s'étend au moins partiellement jusque dans le canal (4).

2. Machine selon la revendication 1, **caractérisée en ce que** le réservoir de cardiotomie (1) et l'oxygénateur (9) forment des unités structurelles dissociables l'une de l'autre qui peuvent en particulier être reliées entre elles par un mécanisme de glissement.

3. Machine selon l'une quelconque des revendications précédentes, **caractérisée en ce qu'**au moins la partie de l'élément échangeur de chaleur (5) qui s'étend dans le canal (4), de préférence l'élément échangeur de chaleur (5), est adaptée sur toute sa longueur du moins sensiblement par sa section transversale extérieure à la section transversale intérieure du canal (4).

4. Machine selon l'une quelconque des revendications précédentes, **caractérisée en ce que** l'extrémité inférieure de l'élément échangeur de chaleur (5) se situe directement en amont ou dans l'orifice où débouche l'entrée de sang (10) de la pompe à sang/de l'oxygénateur (9).

5. Machine selon l'une quelconque des revendications précédentes, **caractérisée en ce que** l'élément échangeur de chaleur (5) est réalisé sous la forme d'un tuyau à double paroi doté d'une conduite coaxiale aller (6) et retour (7) pour le liquide échangeur de chaleur.

6. Machine selon l'une quelconque des revendications précédentes, **caractérisée en ce que** l'élément échangeur de chaleur (5) présente un tuyau central d'afflux et un tuyau d'évacuation s'enroulant en spirale autour du tuyau d'afflux.

7. Machine selon la revendication 5 ou 6, **caractérisée en ce que** le guidage du liquide échangeur de chaleur est tel que le liquide échangeur de chaleur s'écoule dans la partie extérieure de l'élément échangeur de chaleur à contre-courant du sang dans le canal (4).

8. Machine selon l'une quelconque des revendications précédentes, **caractérisée en ce que** l'élément échangeur de chaleur (5) présente sur sa face extérieure des lamelles (5a) qui s'étendent dans le sens longitudinal de l'élément échangeur de chaleur (5).

9. Machine selon l'une quelconque des revendications précédentes, **caractérisée en ce que**, dans le réservoir de cardiotomie (1), au-dessus du canal (4), dans le volume d'urgence (2), est disposé un filtre à sang (8) à travers lequel le sang afflue dans le réservoir de cardiotomie (1).

10. Machine selon la revendication 9, **caractérisée en ce que** le filtre à sang (8) et l'élément échangeur de chaleur (5) sont disposés l'un près de l'autre.

11. Machine selon l'une quelconque des revendications précédentes, **caractérisée en ce que** l'entrée de sang (10) de la pompe à sang peut recevoir du sang avec possibilité de permutation soit depuis la sortie de sang du réservoir de cardiotomie (1), soit en contournant le réservoir de cardiotomie (1) directement depuis une seconde sortie de sang.

12. Machine selon la revendication 11, **caractérisée en ce que**, entre le réservoir de cardiotomie (1) et l'oxygénateur (9), on peut placer un élément intermédiaire dans lequel une permutation entre les sorties de sang peut être provoquée.

13. Machine selon l'une quelconque des revendications précédentes 11 ou 12, **caractérisée en ce qu'**une permutation automatique a lieu en cas de découplage du réservoir de cardiotomie (1).

14. Machine selon l'une quelconque des revendications précédentes, **caractérisée en ce que**, avec une surface de membrane de l'oxygénateur (9) allant de 0,21 à 0,36 m², elle présente un volume de remplissage inférieur à 125 ml, de préférence inférieur à 75 ml, en comptant les tuyaux d'apport et d'évacuation de sang et le volume opératoire du réservoir de cardiotomie (1).
